(19) Europäisches Patentamt
European Patent Office
Office européen des brevets

(11) EP 0 505 440 B1

(12) EUROPEAN PATENT SPECIFICATION

(45) Date of publication and mention
of the grant of the patent:
**22.07.1998 Bulletin 1998/30**

(51) Int Cl.$^6$: **C12N 1/20**, C12N 1/21,
C12N 15/31, C12P 21/00,
A01H 5/00

(21) Application number: **91901185.8**

(22) Date of filing: **12.12.1990**

(86) International application number:
**PCT/US90/07175**

(87) International publication number:
**WO 91/09112 (27.06.1991 Gazette 1991/14)**

(54) **GENETIC CONTROL OF ETHYLENE BIOSYNTHESIS IN PLANTS**

GENETISCHE KONTROLLE DER ÄTHYLEN-BIOSYNTHESE IN PFLANZEN

REGULATION GENETIQUE DE LA BIOSYNTHESE DE L'ETHYLENE CHEZ LES PLANTES

(84) Designated Contracting States:
**AT BE CH DE DK ES FR GB GR IT LI LU NL SE**

(30) Priority: **12.12.1989 US 448095**

(43) Date of publication of application:
**30.09.1992 Bulletin 1992/40**

(60) Divisional application: **98100641.4**

(73) Proprietor: **AGRITOPE, INC.**
**Beaverton, OR 97005 (US)**

(72) Inventors:
 • **FERRO, Adolph, J.**
 **Lake Oswego, OR 97035 (US)**
 • **BESTWICK, Richard, K.**
 **Portland, OR 97223 (US)**
 • **BROWN, Lyle, R.**
 **Corvallis, OR 97330 (US)**

(74) Representative: **VOSSIUS & PARTNER**
**Postfach 86 07 67**
**81634 München (DE)**

(56) References cited:
 **US-A- 4 769 061**

 • **European Journal of Biochemistry, Volume 95,**
 **issued 1979, SPOEREL et al.,**
 **"Colivirus-T3-coded S-adenosyl methionine**
 **hydrolase", pages 227-233, see the entire**
 **document.**

 • **Journal of Bacteriology, Volume 169, No. 8,**
 **issued August 1987, HUGHES et al., "Expression**
 **of the cloned coliphage T3**
 **S-adenosylmethionine hydrolase gene inhibits**
 **DNA methylation and polyamine biosynthesis in**
 **Escherichia coli", pages 3625-3632, see**
 **especially pages 3625 and 3630.**
 • **Cell, Volume 44, issued 31 January 1986,**
 **KOZAK, "Point mutations define a sequence**
 **flanking the AUG initiator codon that modulates**
 **translation by eukaryotic ribosomes", pages**
 **283-292, see the entire document.**
 • **Nucleic Acids Research, Volume 14, No. 24,**
 **issued 1986, MELCHERS et al, "Nucleotide**
 **sequence of the virulence gene virG of the**
 **Agrobacterium tumefaciens octopine Ti**
 **plasmid: significant homology between virG and**
 **the regulatory genes ompR, phoB, and dye of E.**
 **coli." pages 9933-9942, see the entire document.**
 • **Proceedings National Academy of Science, USA,**
 **Volume 83, issued November 1986, WINANS et**
 **al., "A gene essential for Agrobacterium**
 **virulence is homologous to a family of positive**
 **regulatory loci", pages 8278-8282, see the entire**
 **document.**
 • **Plant Molecular Biology, Volume 13, issued**
 **1989, HOOYKAAS, "Transformation of plant**
 **cells via Agrobacterium", pages 327-336, see**
 **especially pages 330-332.**
 • **Nucleic Acids Research, Volume 12 No. 22,**
 **issued 1984, BEVAN, "Bina ry Agrobacterium**
 **vectors for plant transformation" pages**
 **8711-8721, see entire document.**

EP 0 505 440 B1

- Cell, Volume 56, issued 27 January 1989, ZAMBRYSKI et al., "Transfer and Function of T-DNA Genes from Agrobacterium Ti and Ri Plasmids in Plants", pages 193-201, see the entire document.
- Nucleic Acids Research, Volume 15, issued January 1987, HUGHES et al, "Nucleotide sequence and analysis of the coli phage T3 S-adenosylmethionine hydrolase gene and its surrounding ribonuclease III processing sites", pages 717-729, see especially Figure 2.
- Plant Physiology, Volume 73, issued 1983, KUSHAD et, al., "Intermediates in... 5-methylthioribose to methionine in Fruits", pages 257-261, see the entire document, especially Figure 5.
- Plant Physiology, Volume 89, (4 suppl.) issued 1989, KUSHAD et al., "Role of the 5' methylthioadenosine to S-adenosylmethionine salvage pathway in wound ethylene formation", page 113, Abstract 672.
- Biochemical and Biophysical Research Communications, Volume 108, No. 1, issued 16 September 1982, KUSHAD et al., "5-methylthioribose kinase activity in plants", pages 167-173.
- Plant Physiology, Volume 79, issued 1985, KUSHAD et al., "5-methylthioadenosine nucleosidase and 5'-methylthioribose kinase activities and ethylene production during tomato fruit development and ripening", pages 525-529.

**Description**

## BACKGROUND OF THE INVENTION

This invention relates to a method and means of genetic control of ethylene biosynthesis in plants.

Ethylene is a plant hormone influencing many aspects of plant growth and development. This simplest of all unsaturated carbon compounds is a powerful regulator of plant metabolism, acting, and interacting with other plant hormones in trace amounts.

Ethylene promotes senescence in plants, both in selected groups of cells and in whole organs such as fruits, leaves, or flowers. Senescence is the natural, genetically controlled degenerative process which usually leads to death in plants. Even at low concentrations (ethylene is a gas under physiological conditions), ethylene has profound hormonal effects on plants. The effects of ethylene, whether produced by the plant itself or applied exogenously, are numerous, dramatic, and of considerable commercial importance. Among the diverse physiological effects are:

a. Stimulation of ripening in fruits and vegetables
b. Leaf abscission
c. Fading in flowers
d. Flower wilting
e. Leaf yellowing
f. Leaf epinasty

Normally, ethylene production from plant tissue is low. Large quantities of ethylene, however, are produced during ripening and senescence processes. A large amount of ethylene is also produced following trauma caused by chemicals, temperature extremes, water stress, ultraviolet light, insect damage, disease, or mechanical wounding. Ethylene produced by plants under such conditions is referred to as "wound ethylene" or "stress ethylene". In fruits and vegetables, the stimulation of ethylene production by cuts or bruises may be very large and bear considerably on storage effectiveness. Ethylene-induced leaf browning is a common basis for loss in many plants, including lettuce and tobacco. In some tissues, exposure to only a small amount of ethylene may cause an avalanche of ethylene production in adjacent plants or plant tissues such as fresh produce. This autocatalytic effect can be very pronounced and lead to loss of fruit quality during transportation and storage.

The mechanism by which ethylene exerts its effects has become apparent only in the last few years. As judged by numerous data, each of the responses to ethylene involves an ethylene receptor site - a metalloenzyme. The reaction of ethylene with its receptors triggers a cascade of physiological events. Marked increases in the amounts of RNA and protein occur in response to ethylene. The levels of several enzymes have also been shown to increase in response to ethylene, such as cellulase, $\alpha$-amylase, and invertase.

Current technologies that specifically address post-harvest storage life have been in existence for decades and are hampered by such problems as high cost, side effects, and an inability to completely shut off ethylene production. Included in this group are controlled atmosphere (CA) storage, chemical treatment, packaging, and irradiation.

CA facilities slow ethylene biosynthesis through: (1) low temperature, (2) reducing the oxygen level below 3%, and (3) elevating the carbon dioxide level in the storage area to the 3%-5% range. Expensive scrubbers are sometimes added which reduce ethylene already respired to the atmosphere. Drawbacks are that CA facilities are expensive to construct, have a high utility cost, and are unable to completely eliminate ethylene production and side effects. Also, CA storage techniques can only control external ethylene and not that which resides inside the plant tissue. CA storage can also lead to undesirable side effects. Injury can result from high $CO_2$ levels, low $O_2$ levels, or low temperature.

Another approach is to limit ethylene biosynthesis in the plant tissue through chemical treatment. Aminoethoxyvinylglycine (AVG), an analog of the antibiotic rhizobitoxine, is such an inhibitor. Use of the chemical in foods is impossible, however, due to its high toxicity. Silver thiosulfate (STS) is also effective in slowing fruit ripening and flower fading but is also toxic and cannot be used on foods. STS only works with certain flowers and often causes black spotting.

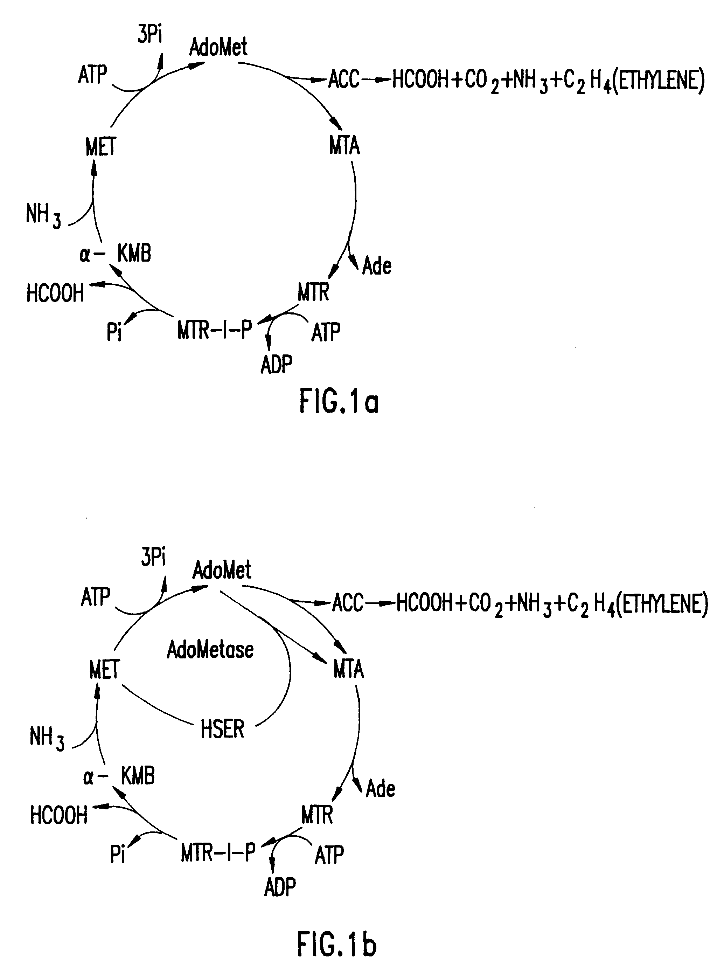

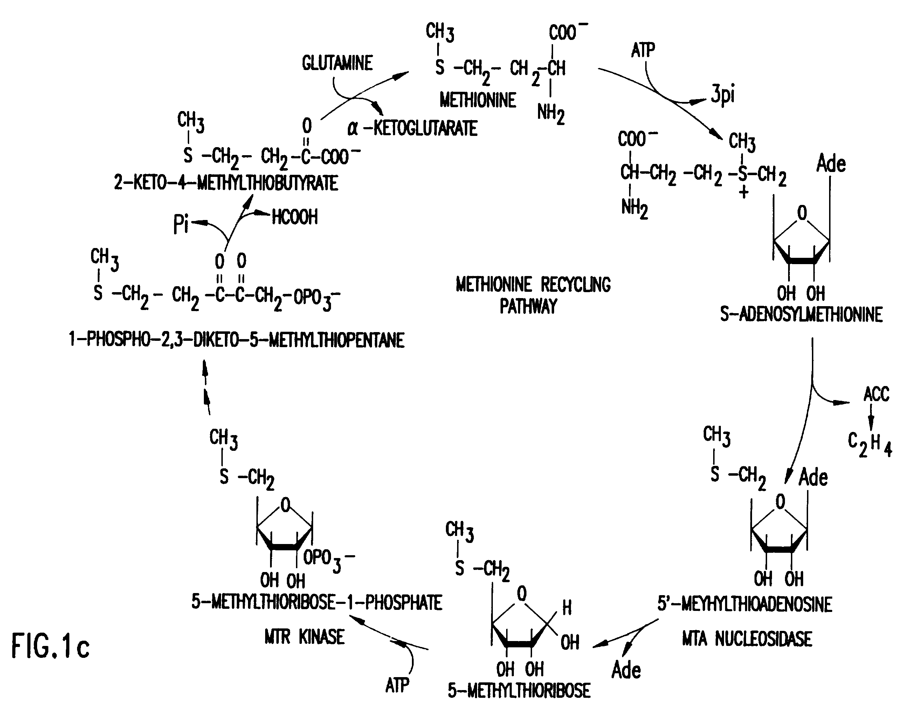

The amino acid methionine has been shown to be a precursor of ethylene in plant tissues. Methionine, however, is not the immediate precursor, but first must be converted to the sulfonium compound S-adenosylmethionine (AdoMet) and, subsequently to 1-aminocyclopropane-1 carboxylic acid (ACC) prior to conversion to ethylene. The following metabolic reactions (also see FIGURE 1) are now accepted for the synthesis of ethylene from methionine under both normal and stress conditions:

$$\text{Methionine} \rightarrow \text{AdoMet} \rightarrow \text{ACC} \rightarrow \text{Ethylene}$$

The system which converts ACC to ethylene appears to be constitutive in most plant tissues with the notable exception of some preclimacteric fruit tissue. ACC synthase catalyzes the degradation of AdoMet to ACC and 5'-methylthioadenosine (MTA). This enzymatic reaction seems to be the rate limiting step in ethylene formation. AdoMet is synthesized via a condensation reaction between methionine and Adenosinetriphosphate (ATP). Attempts at regulating the levels of AdoMet by controlling the rate of AdoMet synthesis have failed, mainly because there appear to be at least three different AdoMet synthesizing enzymes coded by three different genes. In addition, the known biochemical inhibitors of AdoMet synthesis are very toxic to mammalian cells. See S.F. Yang, *et al.,* "Ethylene Biosynthesis and its Regulation in Higher Plants," *Ann.Rev. Plant Physiol,* 35:155-189, 1984; Veen, *et al., SciHortic,* 18:277-286; Sisler, *et al., Plant Physiol,* 63:114-120; and Wang, *et al., Plant Physiol,* 89:434-438.

Although plant tissues are known to maintain a substantial rate of ethylene production for extended periods, their methionine levels have been shown to be very low. To continue to produce ethylene, the sulfur contained in MTA must be recycled back into methionine so as to provide an adequate supply of methionine for continual ethylene production. This pathway has been recently shown to exist in plant tissue (see FIGURE 1c). See also S.F. Yang, *et al.,* "Ethylene Biosynthesis and its Regulation in Higher Plants," *Ann.Rev. Plant Physiol,* 35:155-189, 1984. The degradation of MTA has added significance in light of the finding that MTA is a potent inhibitor of ACC synthase. It should be noted that this pathway merely maintains a methionine supply for ethylene biosynthesis, but does not result in a net increase in methionine synthesis.

An enzyme encoded by the *E. Coli* bacteriophage T3 hydrolyzes S-adenosylmethionine (AdoMet) to homoserine and 5'-methylthioadenosine (MTA). This enzyme is known by either its recommended name, AdoMet hydrolase (AdoMetase), or by its other name, S-adenosylmethionine cleaving enzyme (SAMase). See Studier, *et al.,* "SAMase Gene of Bacteriophage T3 is Responsible for Overcoming Host Restriction," *Journal of Virology,* 19:135-145, 1976. Both products of the reaction are recycled to methionine; MTA as previously shown (FIGURE 1) and homoserine via a metabolism pathway known to exist in plant tissues. The AdoMetase gene has been identified, isolated, cloned, and sequenced. J.A. Hughes, *et al.,* "Expression of the Cloned Coliphage T3 S-adenosylmethionine Gene Inhibits DNA Methylation and Poly Amine Biosynthesis in *Escherichia coli", J.Bact.,* 169:3625-3632, 1987 and J.A. Hughes, *et al.,* "Nucleotide Sequence and Analysis of the Coliphage T3 S-adenosylmethionine Hydrolase Gene and its Surrounding Ribonuclease III Processing Sites", *Nuc. Acids Res.,* 15:717-729, 1987. The gene contains two inframe reading sequences that specify polypeptides of 17105 and 13978 daltons. Both polypeptides terminate at the same ochre codon. This results in the 14 kd polypeptide being Identical to 82% of the 17kd polypeptide starting at the carboxyl end of the longer polypeptide. Both polypeptides are present in partially purified preparations of active AdoMetase from T3 bacteriophage infected cells and from *E. Coli* expressing the cloned gene. JA. Hughes, *et al., "Nucleotide* Sequence and Analysis of the Coliphage T3 S-adenosylmethionine Hydrolase Gene and its Surrounding Ribonuclease III Processing Sites," *Nuc. Acids Res.,* 15:717-729, 1987 and F.W. Studier, *et al.,* "SAMase Gene of Bacteriophage T3 is Responsible for Overcoming Host Restriction," *J.Virol.,* 19:136-145, 1976.

Other bacteriophages that encode the AdoMetase or SAMase genes are coliphage BA14, *Klebsiella* phage K11, and *Serratia* phage IV. See H. Mertens, *et al.,* "Collphage BA14: a New Relative of Phage T7; *J.Gen. Virol.,* 62:331-441, 1982; R. Hausmann, *The Bacteriophages,* 1:279-283, 1988, R. Calender (ed.), Plenum Press, New York; and K.H. Korsten, *et al.,* "The Strategy of Infection as a Criterion for Phylogenetic Relationships of Non-Coli Phages Morphologically Similar to Phage T7," *J.Gen. Virol.,* 43:57-73, 1979.

## SUMMARY OF THE INVENTION

The present invention relates to a vector useful for transformation of a plant host comprising a first DNA sequence containing a gene useful for genetic selection in plant cells, where the first DNA sequence is regulated by a plant promoter effective to allow expression of the sequence in a plant host. The vector also includes a second DNA sequence encoding an S-adenosylmethionine hydrolase enzyme (i. e., SAMase) which hydrolyses S-adenosylmethionine to homoserine and 5'-methylthioadenosine. The second DNA sequence is flanked by a plant promoter on one side and a polyA sequence on the other side effective to allow expression of the sequence in a plant host. The DNA codons may be obtained from cDNA or genomic DNA, or may be prepared by DNA synthesis techniques.

According to one embodiment of the invention, the plant promoter flanking the second DNA sequence is capable of regulating tissue specific, temperature dependent, or light dependent expression of the second DNA sequence.

In an alternative embodiment, the plant promoter flanking the second DNA sequence is a constitutive expression promoter. Constitutive expression promoters for use in the present invention include a nopaline synthetase promoter and a Cauliflower Mosaic Virus promoter.

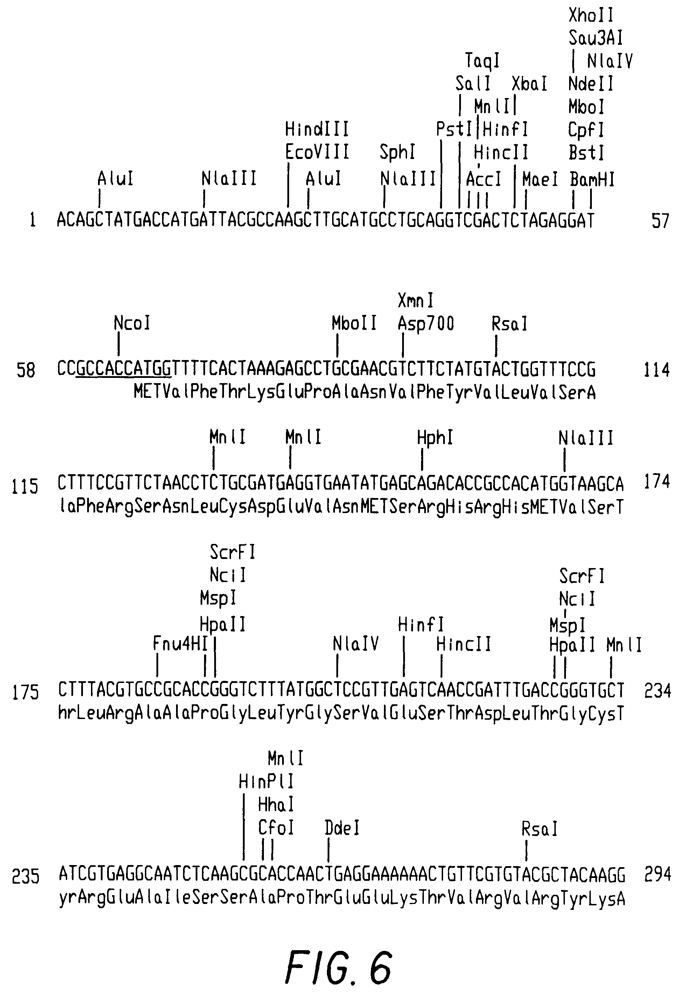

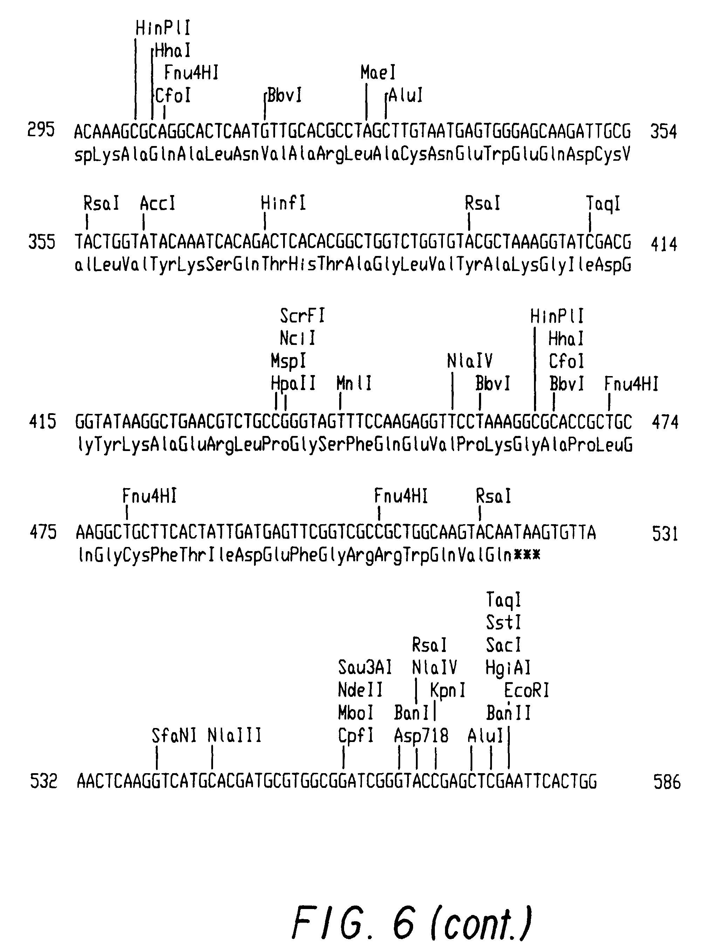

The vector of the present invention contains a second DNA sequence encoding an S-adenosylmethionine hydrolase enzyme. This second DNA sequence may be obtained from a number of sources, and in one embodiment, is from a bacteriophage selected from the group consisting of *Escherichia coli* bacteriophage T3, coliphage BA14, *Klebsiella* phage K11, and *Seratti* phage IV. According to a specific embodiment of the invention, the second DNA sequence encodes the protein sequence presented in Figure 6.

The vector of the invention also contains a gene useful for genetic selection in plant cells. In one embodiment, a vector of the invention is composed of a gene which confers kanamycin resistance.

In another aspect, the invention provides a method for reducing ethylene biosynthesis in plant cells. In practicing the method, plant host cells are transformed with a vector comprising a first DNA sequence containing a gene useful for genetic selection in plant cells, where the first DNA sequence is regulated by a plant promoter effective to allow expression of the sequence in a plant host. The vector also includes a second DNA sequence encoding an S-adenosylmethionine hydrolase enzyme (*i.e.,* SAMase) which hydrolyses S-adenosylmethionine to homoserine and 5'-methylthioadenosine. The second DNA sequence is flanked by a plant promoter on one side and a polyA sequence on the other side effective to allow expression of the sequence in a plant host.

In one embodiment, transformation of a plant host is carried out by a direct transformation methodology. Direct transformation methodologies for use in the present method include *Agrobacterium*-mediated binary vector transformation, electroporation, microinjection, and microprojectile bombardment.

According to one embodiment, the invention provides a method for reducing ethylene biosynthesis in *Nicotiana tabacum* L. cv. Wisconsin, wherein the method includes transforming *Nicotiana tabacum* L. cv Wisconsin plant host cells.

According to yet another embodiment, the invention provides a method for reducing ethylene biosynthesis in tomato, in which tomato plant host cells are transformed with a vector of the invention.

The present invention further provides a transgenic plant cell containing a DNA sequence which encodes and expresses a S-adenosylmethionine hydrolase enzyme. According to a specific embodiment of the invention, the plant cell is obtained from a fruit, vegetable or flower producing plant, and may further comprise a gene useful for genetic selection in plant cells.

## BRIEF DESCRIPTION OF THE DRAWINGS

The invention itself, as well as additional advantages and features thereof, will be more readily and comprehensively understood from the following detailed description of the preferred inventive embodiments, such description making reference to the appended sheets of drawings, wherein:

FIGURE 1a schematically represents the ethylene biosynthetic pathway in plant tissue;

FIGURE 1b schematically represents the ethylene biosynthetic pathway with AdoMetase;

FIGURE 1c schematically represents the methionine recycling pathway;

FIGURE 2 schematically represents the construction of pBI121-AdoMetase;

FIGURE 3 schematically represents the genetic engineering of the AdoMetase gene;

FIGURE 4 schematically represents the alternative construction of pGA482-NOS-SAM;

FIGURE 5 indicates SAMase activity in transgenic plants; and

FIGURE 6 indicates that part of the nucleotide sequence of pUC19SAM-K that encodes the AdoMetase gene with the modified 5' end.

## DETAILED DESCRIPTION OF THE INVENTION

AdoMetase is normally not present in plant tissues. The AdoMetase gene codes for a protein having a very unusual enzymatic activity. Bacteriophage T3, Coliphage BA14, *Klebsiella* phage K11, and *Serratia* phage IV are the only known sources of a gene encoding that activity. The presence of the AdoMetase gene and the expression of AdoMetase in transgenic plants lowers AdoMet levels. This effect is shown schematically in FIGURE 1b. Since AdoMet is the sole precursor for ethylene biosynthesis, its reduced availability causes a corresponding decrease in ethylene biosynthesis. Furthermore, the hydrolysis of AdoMet by AdoMetase generates MTA which is an inhibitor of ACC synthase, a principle enzyme in the biosynthesis of ethylene by plants. The net effect is twofold, a reduction in precursor availability and a direct inhibition of ethylene biosynthesis. The current construction of transgenic plants containing at least one copy of the T3 AdoMetase gene by use of the *Agrobacterium* transfer systems allow for construction of plants that will control ethylene biosynthesis under restricted conditions. Thus, the present invention combines expertise from two very different fields of study, bacteriophage biochemistry, and plant biochemistry. This invention will result in fruits, vegetables, and flowers which have been modified internally to improve shelf life and preservation qualities.

In one embodiment, the present invention provides a binary vector system useful for the transformation of a plant host. The binary vector system includes a "T-DNA less" Ti plasmid, a broad host-range plasmid containing T-DNA borders, and a gene useful for genetic selection under plant promoter control. The vector also includes a DNA insert comprising codons for a functional heterologous polypeptide having AdoMetase activity, or a heterologous polypeptide having substantially the same biological activity as AdoMetase. The heterologous gene is flanked by a plant promoter on one side and a polyA signal sequence on the other side. The result is the transformed plant host is capable of expressing the heterologous polypeptide under the control of the control region.

In another embodiment, the present invention provides a tripartite vector system useful for transformation of a plant host. The tripartite vector system includes (a) a "T-DNA less" Ti plasmid, (b) a broad host-range P incompatibility group plasmid containing a cloned virG gene, and (c) a broad host-range plasmid containing T-DNA borders, and a gene useful for genetic selection under plant promoter control. The vector also includes a DNA insert comprising codons for a functional heterologous polypeptide having AdoMetase activity, or a heterologous polypeptide having substantially the same biological activity as AdoMetase activity, or functional derivatives thereof. The heterologous gene is flanked by a plant promoter on one side and a polyA signal sequence on the other side. The result is the transformed plant host is capable of expressing the heterologous polypeptide under the control of the promoter region. See *Cell,* 56: 193-201.

Specifically, such a vector has been constructed using a strain of *Agrobacterium* PC2760 containing a T-DNA-less derivative of pTiA6NC. To this transfer system has been added a plasmid pVK102 containing a plasmid pTiBo542 virG gene insert. This insert enhances the transfer of the DNA containing T-DNA borders that are contained on a third plasmid. The third plasmid may be pGA 482-Sam-K or pBI 121-AdoMetase.

In summary, such a tripartite transfer system is provided by three plasmids contained in the same bacterial strain, *e.g., Agrobacterium* PC2760. The first plasmid contains all of the genetic information needed to transfer genes to plants with the exception of the T-DNA borders. The second plasmid contains extra copies of one of the virulence genes which enhances the transfer process. The third plasmid contains the DNA to be delivered to the plants cells *(e.g.,* gene encoding SAMase) engineered between two T-DNA borders. This system differs from those published in that it uses three plasmids and a mixture of virulence gene products (ones from pTiA6NC and pTiBo542) to achieve efficient transfer to a broad variety of plants.

## DESCRIPTION OF THE PREFERRED EMBODIMENT

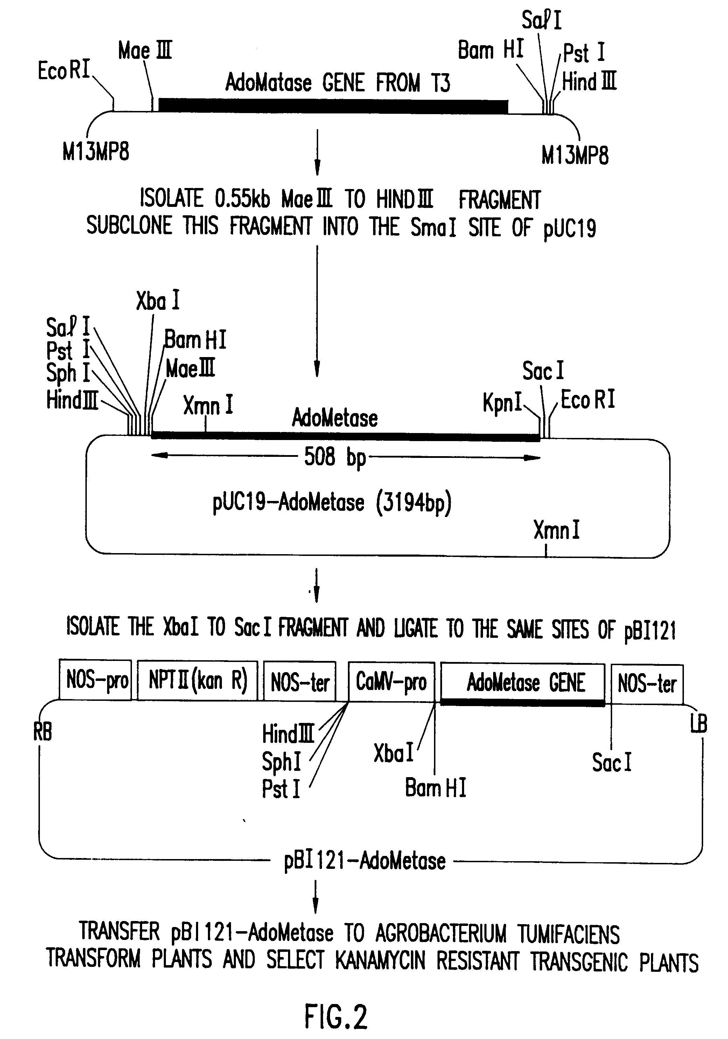

The plant vector system consists of the following. To transfer the AdoMetase gene into plants, a binary *Agrobacterium tumefaciens* system is preferably used. The *Agrobacterium* strain PC2760 containing a "T-DNA less" Ti plasmid and a broad host-range plasmid containing T-DNA borders, a selective kanamycin gene under plant promoter control, and the AdoMetase gene flanked by a plant promoter on one side, and a polyA signal sequence on the other side is constructed as shown in FIGURE 2 and described in the following examples.

It will be appreciated that the AdoMetase gene can be isolated from more than one bacteriophage. Different bacteriophages may be expected to contain AdoMetase genes with variations in their DNA sequences. Furthermore, the amino acid sequence of AdoMetase may be modified by genetic techniques to produce enzymes with altered biological activities. An increase in the biological activity could permit the use of lower amounts of the enzyme to control ethylene biosynthesis in plants. Modifications of the sequence of the AdoMetase gene are within the scope of the present invention.

## EXAMPLE 1

The source of the AdoMetase gene is obtained and manipulated as follows. The AdoMetase gene has been identified on an AluI-HaeIII restriction fragment from purified T3 DNA (J.A. Hughes, *et al.,* "Expression of the Cloned Coliphage T3 S-adenosylmethionine Gene Inhibits DNA Methylation and Poly Amine Biosynthesis in *Escherichia Coli,"* *J.Bact.,* 169:3625-3632, 1987). Bacteriophage T3 is available under ATCC No. 11303-B3. This DNA fragment was first cloned into the bacteriophage M13 MP8 vector (Pharmacia LKB Biotechnology, Inc.). A Mae 111 to Bam fragment is then subcloned into the pUC19 plasmid vector (Pharmacia) to produce pUC19-AdoMetase (pUC19-SAMase), transformed into *E. Coli* and used as a source of DNA for further construction experiments and for DNA sequence determination. The Mae 111 site is used as the 5' terminus of the AdoMetase gene fragment since it is only 10 base pairs upstream from the initiation codon for the gene. As shown in FIGURE 2, pUC19-AdoMetase is used as the source of the AdoMetase gene for insertion into an *Agrobacterium tumefaciens* vector as described below.

The parent vector, pBI121, is obtained commercially from Clontech Laboratories, Inc. The plant promoter upstream of the AdoMetase gene sequence can be varied to obtain tissue specific expression, temperature dependent expression, low or high level constitutive expression, hormone-induced expression, or light dependent expression in the transgenic plants. In the following example, the promoter is the constitutive Cauliflower Mosaic Virus (CaMV) promoter (Pharmacia).

## EXAMPLE 2

The following is an example of the construction and transformation with PC2760/pBl121-AdoMetase. The pUC19-AdoMetase plasmid is digested with Xba I and Sac I to produce a 520bp fragment encoding the entire AdoMetase gene. The DNA fragment is purified by agarose gel electrophoresis followed by electrolution. The vector, pBl121, is also digested with Xba I and Sac I and purified by the same method as described above. The two fragments are ligated together and the resultant plasmid named pBl121-AdoMetase. PBl121-AdoMetase is introduced into *Agrobacterium* using a direct transformation method. *Agrobacterium tumefaciens* PC2760 is deposited with the American Type Culture Collection, Rockville, Maryland, under accession number ATCC 68111. *Agrobacterium tumefaciens* strain PC2760 is grown to mid log phase (OD 600 0.5 to 1.0) in YEP media (10g yeast extract, 10g peptone, and 5g NaCl per liter). After chilling on ice, 50mls of these cells are pelleted, resuspended in 1ml of ice cold 20mM $CaCl_2$ and split into 1ml aliquots. One $\mu$g of pBl121-AdoMetase is added to one of the aliquots and incubated on ice for 30 minutes, frozen in liquid nitrogen and thawed at 37°C for 5 minutes. One ml of YEP media is added and incubated at 28°C for 2 hours. The cells are pelleted and resuspended in 50$\mu$l of YEP, then plated on YEP agar plates containing 20$\mu$g/ml kanamycin. Kanamycin-resistant transformed colonies appear within 2 days.

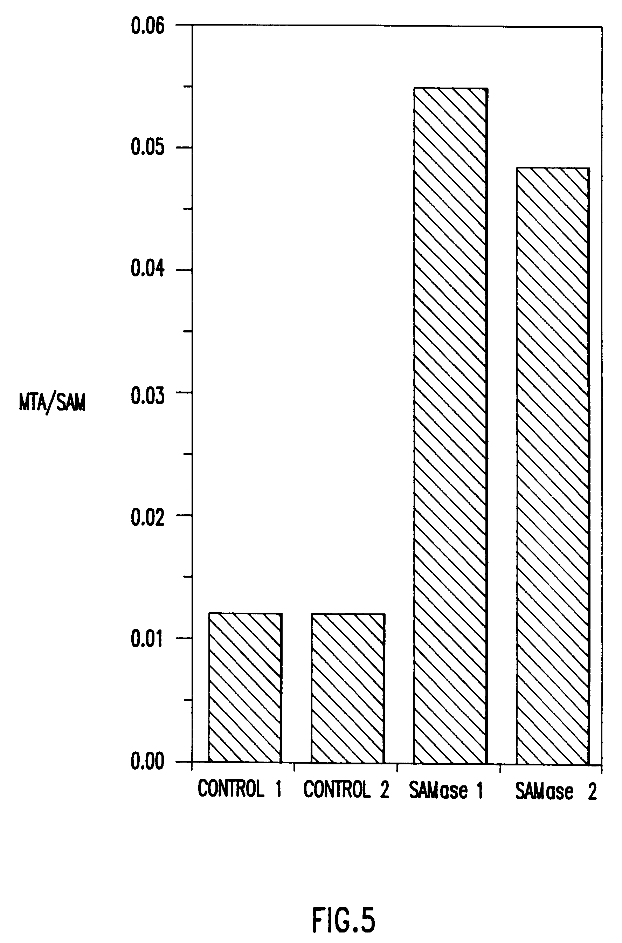

A PC2760 clone containing these plasmids is named PC2760/pBl121-AdoMetase and was used to transform leaf discs obtained from Nicotiana tabacum L. cv. Wisconsin by the following direct method. A tobacco leaf is washed once in 95% ethanol for 10 seconds, once in 10% bleach, 0.1% Tween-20 for 20 minutes, four times in water, cut into 5mm discs, and finally placed in a 10ml overnight culture of PC2760/pBl121 for 30 minutes. The leaf discs are then placed on Murishegee and Sckoog callus forming medium for 1 day. The discs are then soaked in 500$\mu$g/ml cefatoxamine for 1 hour and placed on Tobacco callus-forming media containing 200$\mu$g/ml carbenicillin for 3 days. The discs are then transferred to the same medium containing an additional 100$\mu$g/ml kanamycin. Kanamycin-resistant tobacco callus is selected using standard techniques. The regeneration of plants from calli is a known art. Protocols vary with each plant species and specific parameters can be easily determined by one skilled in the art. Plant tissues derived from this callus are shown to contain the AdoMetase gene using DNA-dot blots and Southern blots. Transcription of this gene Is demonstrated by extracting RNA from leaf tissue and performing Northern blots. Both Southern and Northern blots are probed with a radioactively-labeled AdoMetase gene fragment from pUC1 9-AdoMetase. The presence of AdoMetase enzyme is confirmed by making crude extracts from leaf tissue and performing AdoMetase assays as previously described and as demonstrated in FIGURE 5 where extracts of transgenic plants were analyzed for enzymatic activity based on the ratio of 5'methylthioadenosine to S-adenosylmethionine. Also demonstrated is the effect of naphthaleneacetic acid (NAA), a plant hormone which stimulates ethylene production, on control tissues versus transgenic plant tissue In terms of ethylene evolution In tobacco leaf discs after 40 hours of culture. The transgenic tissue Is designated Nt-BOB. The transgenic plant shows a marked decrease in ethylene evolution as shown in TABLE 1.

TABLE 1

| The Effect of NAA on Ethylene Evolution of AdoMetase Transformed Tobacco Leaf Discs After 40 Hours of Culture | | |
|---|---|---|
| Tissue | NAA (mM) | Ethylene (nmol/g/40 h) |
| Nt-control | 0.00 | 1.90 |
| | 0.01 | 10.01 |
| | 1.00 | 75.47 |
| Nt-BOB | 0.00 | 0.61 |
| | 0.01 | 5.17 |
| | 1.00 | 13.01 |

As seen in FIGURE 1, the formation of ACC is a rate limiting step for production of ethylene in plant tissues. S.F. Yang, *et al.,* "Ethylene Biosynthesis and Its Regulation in Higher Plants," *Ann.Rev. Plant Physiol,* 35:155-189, 1984. Various other methods may be employed to elicit transformation of the plant host, such as electroporation, microinjection, and microprojectile bombardment. These methods are well known in the art and detailed in the following representative references. T.M. Klein, *et al.,* "Stable Genetic Transformation of Intact Nicotiana Cells by the Particle Bombardment Process," *Proc.Natl.Acad.Sci. USA,* Washington, D.C.: The Academy, Nov. 1988, vol. 85, Issue 22, pages 8502-8505, ill.; B.LA. Miki, *et al.,* "Microinjection: An Experimental Tool for Studying and Modifying Plant Cells," *Plant*

*DNA Infectious Agents,* edited by Th. Hohn and J. Schell, Wien: Springer-Verlag, c1987, pages 249-265, ill.; C. Bellini, *et al.,* "Transgenic Plants of Lettuce (Lactuca Sativa) Obtained Through Electroporation of Protoplasts," *Bio/Technol,* New York, NY: Nature Publishing Co., May 1989, Vol. 7, Issue 5, pages 503-508, ill. An analogous PC2760/pBl121-AdoMetase clone containing the virG gene constituting a tripartite vector system is also employed to transform leaf discs by the method described above.

The present method is applicable to all higher plants, and particularly relevant for use with economically significant food crops and ornamentals. The following list of plant species to which the present method may be applied is representative of the wide range of applications, but is by no means limiting thereto.

Food crops:

Allium cepa (onion)
Allium sativum (garlic)
Ananas comosus (pineapple)
Ananas sativus (pineapple)
Apium graveolens (celery)
Asparagus officinalis (asparagus)
Beta vulgaris (red and sugar beets)
Brassica oleracea (cole crops)
Capsicum annum (peppers)
Capsicum frutescens (peppers)
Carica candamarcensis (papaya)
Carica cauliflora (papaya)
Carica papaya (papaya)
Cichorium endivia (endive)
Citrullus lanatus (watermelon)
Citrullus sp. (melons)
Citrulius vulgaris (watermelon)
Cucumis melo (cantaloupe)
Cucumis sativus (cucumber)
Cynara scolymus (Globe artichoke)
Daucus carota (carrots)
Ficus carica (figs)
Fragaria sp. (strawberry)
Fragaria x ananassa (strawberry)
Lactuca sativa (lettuce)
Lycopersicon esculentum (tomato)
Malus pumila (apple)
Malus sylvestris (apple)
Musa acuminata (banana)
Musa cavendishii (banana)
Musa sp. (banana)
Olea europaea (olive)
Passiflora edulis (passion fruit)
Persea americana (avocado)
Phaseolus vulgaris (bean)
Phoenix dactylifera (date palm)
Pisum sativum (pea)
Prunus avium (cherry)
Prunus domestica (plum)
Prunus institia (plum)
Prunus mariana (prunus rootstock)
Prunus pandora (cherry)
Prunus persica (peach)
Prunus sp. (apricot, nectarines)
Punica granatum (pomegranate)
Pyris communis (pear)
Rubus idaeus (raspberry)
Rubus sp. (cane berries)

Rubus ursinus (raspberry)
Solanum melongena (eggplant)
Solanum tuberosum (potato)
Spinacla oleracea (spinach)
Vaccinium elliottii (blueberry)
Vaccinium macrocarpon (cranberry)
Vaccinium sp. (blueberry)
Vitis labruscana (concord grape)
Vitis rupestris (grape)
Vitis sp. (grapes)
Vitis vinifera (wine grapes)
Zea mays (corn)

Ornamentals:

Antirrhinum majus (snapdragon)
Chrysanthemum morifolium
Delphinium cardinale
Delphinium elatum
Delphinium nudicaule
Dianthus caryophyllus (carnation)
Euphorbia pulcherrima (poinsettia)
Fuchsia hybrida
Gerbera jamesonii (daisy)
Gladiolus grandiflorus
Gladiolus hortulans
Homerocallis sp. (day lilly)
Iris hollandica
Iris sp.
Lilium sp. (lily)
Narcissus sp. (daffodil/narcissus)
Pelargonium hortorum (geranium)
Pelargonium peltatum (geranium)
Pelargonium sp.
Pelargonium zonale (geranium)
Petunia axillaris
Petunia hybrida
Petunia inflata
Petunia parodii
Petunia parviflore
Petunia sp.
Petunia tricuspidata
Rhododendron simsii (azalea)
Rhododendron sp.
Rosa canina
Rosa chinensis
Rosa damascena
Rosa hybrida
Rosa manetti
Rosa nitida
Rosa multiflora
Rosa sp.
Saintpaulia ionantha (african violet)
Tullpa gesneriana (tulip)

Orchids:

Arachnis sp.

Cattleya sp.
Cymbidium sp.
Dendrobium sp.
Oncidium sp.
Paphiopedilum sp.
Vanda sp.

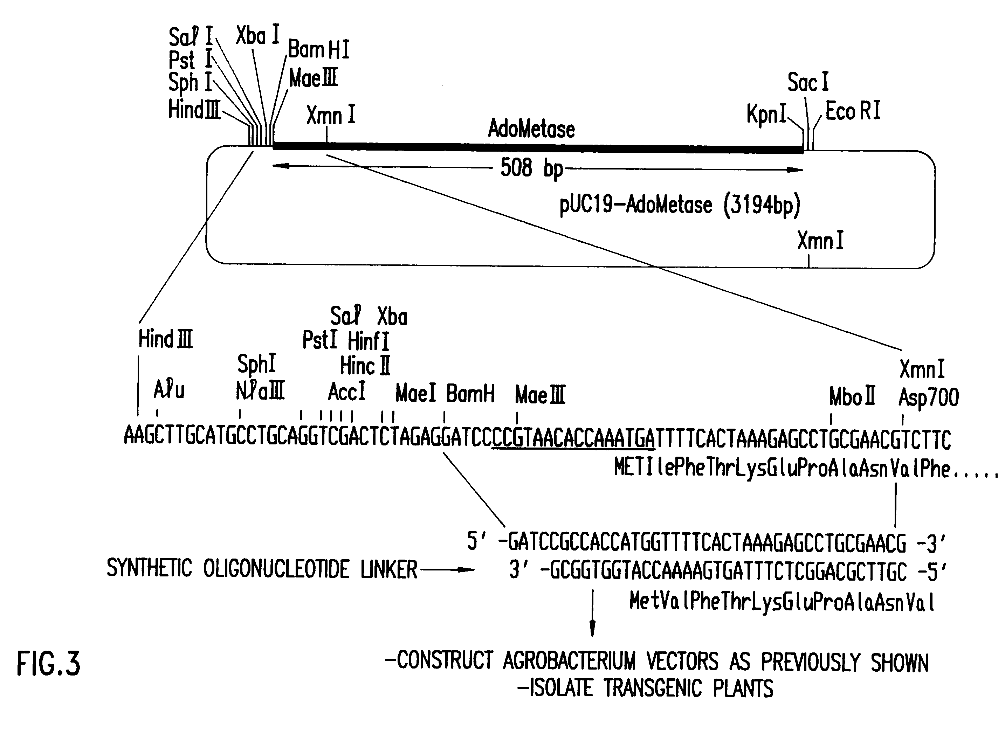

The AdoMetase gene is genetically engineered further to achieve a preferred sequence. Analysis of the AdoMetase gene sequence indicated a less than optimal DNA sequence surrounding the initiation codon of the gene. According to the studies of M. Kozak, "At least Six Nucleotides Preceding the AUG Initiator Codon Enhance Translation in Mammalian Cells," *J.Mol.Bio.,* 196:947-950, 1987, a consensus initiation sequence for eucaryotic mRNAs exists which allows for efficient translation. The AdoMetase gene is genetically engineered to change the AdoMetase initiation sequence to the consensus Kozak sequence. The changes made to the DNA sequence are shown in FIGURE 3 and carried out as follows.

## EXAMPLE 3

The plasmid pUC1 9-AdoMetase is digested with Xmn I and Bam HI and the 1.9kb and 1.3kb fragments purified by electrolution after agarose gel electrophoresis. A double stranded synthetic oligonucleotide linker having the sequence indicated in FIGURE 3 is ligated to the 1.9kb fragment and this ligated DNA subjected to Xmn I digestion to remove excess linkers. The linkered 1.9kb fragment is then repurified by electrophoresis on low melting temperature agarose and ligated to the 1.3kb fragment to form the plasmid pUC19 SAM-K. The altered gene region is subjected to DNA sequence analysis and shown to contain the expected DNA sequence as shown in FIGURE 6. This gene is named SAM-K and used to construct additional plant expression vectors. A pBl121-SAM-K (PC2760/pBl121-SAM-K) construction is created and transferred into tobacco using the approach described above in EXAMPLE 1. The plasmid DNA can also be used to directly transform the plant host via electroporation, microinjection, or microprojectile bombardment.

## EXAMPLE 4

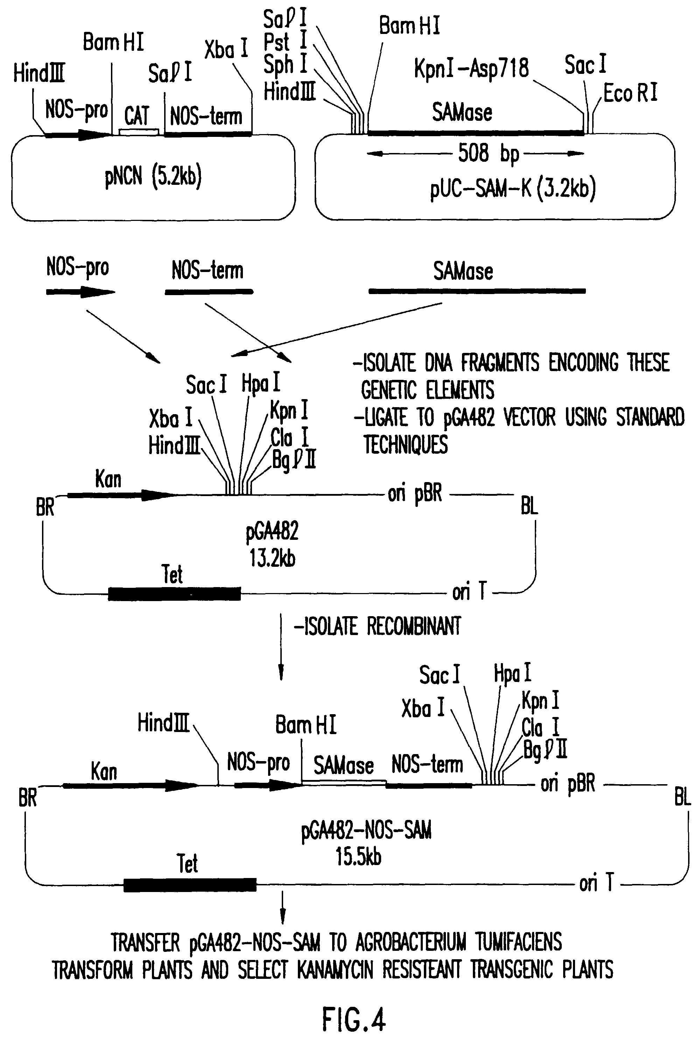

The following Example discloses an alternative construction. FIGURE 4 describes the construction of vector pGA482-NOS-SAM which is analogous to pBl121-AdoMetase or pBl121-SAM-K above. In this construction, a different promoter is employed as well as different parental plasmids. The parental plasmids are pGA482 and pNCN obtained from Pharmacia. The promoter used is the constitutive nopaline synthetase promoter (NOS-pro and NOS-term).

Using standard techniques, the DNA fragments NOS-pro and NOS-term are isolated from pNCN, and the SAMase fragment coding for the altered enzyme is isolated from pUC-SAM-K. The fragments are ligated into pGA482 at the appropriate restriction sites as indicated in the figure with the NOS-pro and NOS-term sequences flanking the SAMase fragment. The plasmid GA482-NOS-SAM is transferred to *A. tumefaciens* (PC2760/GA482-NOS-SAM) and used to transform plants as above.

While there is shown and described present preferred embodiments of the invention, it is to be distinctly understood that the invention is not limited thereto, but may be otherwise variously embodied and practiced within the scope of the following claims.

## Claims

1. A vector useful for transformation of a plant host, said vector comprising:

   a first DNA sequence containing a gene useful for genetic selection in plant cells, where said first DNA sequence is regulated by a plant promoter effective to allow expression of the sequence in a plant host, and where said vector further comprises
   a second DNA sequence which (i) is flanked by a plant promoter on one side and a poly A sequence on the other side effective to allow expression of the sequence in a plant host, and (ii) encodes a S-adenosylmethionine hydrolase enzyme which hydrolyses S-adenosylmethionine to homoserine and 5'-methylthioadenosine.

2. The vector of claim 1, wherein the plant promoter flanking the second DNA sequence provides tissue specific, temperature dependent, or light dependent expression of the second DNA sequence.

3. The vector of claim 1 or 2, where said second DNA sequence encodes the protein sequence presented in Figure 6.

4. The vector of claim 1 or 2, where said second DNA sequence is derived from a bacteriophage selected from the group consisting of *Escherichia coli* bacteriophage T3, coliphage BA14, *Klebsiella* phage K11, and *Seratti* phage IV.

5. The vector of any one of claims 1 to 4, wherein said gene useful for genetic selection in plant cells confers kanamycin resistance.

6. The vector of any one of claims 1 to 5, wherein the plant promoter flanking the second DNA sequence is a constitutive nopaline synthetase promoter.

7. The vector of any one of claims 1 to 5, wherein said plant promoter flanking the second DNA sequence is a constitutive expression promoter.

8. The vector of claim 7, wherein said constitutive expression promoter is a Cauliflower Mosaic Virus promoter.

9. A method for reducing ethylene biosynthesis in plant cells, comprising:
transforming plant host cells with a vector of any of claims 1 to 8, wherein the transformed plant host cells are capable of expressing said enzyme.

10. The method of claim 9, wherein the transformation of a plant host is carried out by a direct transformation methodology selected from the group consisting of *Agrobacterium*-mediated binary vector transformation, electroporation, microinjection, and microprojectile bombardment.

11. The method of claims 9 or 10, wherein said plant host is *Nicotiania tabacum* L. cv. Wisconsin.

12. The method of claims 9 or 10, wherein said plant host is a tomato.

13. A transgenic plant cell containing a DNA sequence which encodes and expresses a S-adenosylmethionine hydrolase enzyme, where said enzyme can hydrolyse S-adenosylmethionine to homoserine and 5'-methylthioadenosine.

14. A transgenic plant cell of claim 13, further comprising a gene useful for genetic selection in plant cells.

15. A transgenic plant cell of claim 13 or 14, where said plant cell is obtained from a fruit, vegetable or flower producing plant.


**Patentansprüche**

1. Vektor, verwendbar zur Transformation eines Pflanzenwirts, umfassend:

eine erste DNA-Sequenz, die ein Gen enthält, das zur genetischen Selektion in Pflanzenzellen geeignet ist, wobei die erste DNA-Sequenz durch einen pflanzlichen Promotor reguliert wird, der die Expression dieser Sequenz in einem pflanzlichen Wirt erlaubt, und wobei der Vektor weiterhin umfaßt:
eine zweite DNA-Sequenz, die (i) von einem Pflanzenpromotor an einer Seite und einer Poly-A Sequenz an der anderen Seite flankiert wird, die die Expression der Sequenz in einem Pflanzenwirt erlauben, und (ii) ein S-Adenosylmethionin-Hydrolaseenzym codiert, das S-Adenosylmethionin zu Homoserin und 5'-Methylthioadenosin hydrolysiert.

2. Vektor nach Anspruch 1, wobei der die zweite DNA-Sequenz flankierende Pflanzenpromotor die gewebsspezifische, temperaturabhängige oder lichtabhängige Expression der zweiten DNA-Sequenz erlaubt.

3. Vektor nach Anspruch 1 oder 2, wobei die zweite DNA-Sequenz die in Figur 6 dargestellte Proteinsequenz codiert.

4. Vektor nach Anspruch 1 oder 2, wobei die zweite DNA-Sequenz von einem Bakteriophagen stammt, der ausgewählt ist aus der Gruppe bestehend aus *Escherichia coli* Bakteriophage T3, Coliphage BA14, *Klebsiella-Phage* K11 und *Seratti* Phage IV.

**5.** Vektor nach einem der Ansprüche 1 bis 4, wobei das Gen, das zur genetischen Selektion in Pflanzenzellen geeignet ist, Kanamycinresistenz verleiht.

**6.** Vektor nach einem der Ansprüche 1 bis 5, wobei der die zweite DNA-Sequenz flankierende Pflanzenpromotor ein konstitutiver Nopalinsynthetase Promotor ist.

**7.** Vektor nach einem der Ansprüche 1 bis 5, wobei der die zweite DNA-Sequenz flankierende Pflanzenpromotor ein konstitutiver Expressionspromotor ist.

**8.** Vektor nach Anspruch 7, wobei der konstitutive Expressionspromotor ein Blumenkohlmosaikvirus-Promotor ist.

**9.** Verfahren zur Reduzierung der Ethylenbiosynthese in Pflanzenzellen, umfassend: die Transformation von Pflanzenwirtszellen mit einem Vektor nach einem der Ansprüche 1 bis 8, wobei die transformierten Pflanzenwirtszellen zur Expression des Enzyms fähig sind.

**10.** Verfahren nach Anspruch 9, wobei die Transformation eines Pflanzenwirts mit Hilfe einer direkten Transformationsmethode ausgeführt wird, die ausgewählt ist aus einer Gruppe bestehend aus *Agrobakterium*-vermittelter binärer Vektortransformation, Elektroporation, Mikroinjektion und Mikroprojektilbeschuß.

**11.** Verfahren nach Anspruch 9 oder 10, wobei der Pflanzenwirt *Nicotiania tabacum* L. cv. Wisconsin ist.

**12.** Verfahren nach Anspruch 9 oder 10, wobei der Pflanzenwirt eine Tomate ist.

**13.** Transgene Pflanzenzelle, enthaltend eine DNA Sequenz, die ein S-Adenosylmethionin-Hydrolaseenzym codiert und exprimiert, wobei das Enzym S-Adenosylmethionin zu Homoserin und 5'-Methylthioadenosin hydrolysieren kann.

**14.** Transgene Pflanzenzelle nach Anspruch 13, die weiterhin ein Gen enthält, das zur genetischen Selektion in Pflanzenzellen geeignet ist.

**15.** Transgene Pflanzenzelle nach Anspruch 13 oder 14, wobei die Pflanzenzelle von einer Früchte, Gemüse, oder Blüten produzierenden Pflanze gewonnen wird.

**Revendications**

**1.** Vecteur utilisable pour une transformation d'un hôte de type plante, ce vecteur comprenant: une première séquence d'ADN contenant un gène utilisable pour une sélection génétique dans des cellules de plante, où ladite première séquence d'ADN est réglée par un promoteur de plante efficace pour permettre une expression de la séquence dans un hôte de type plante, et où le vecteur comprend en outre une seconde séquence d'ADN qui (i) est bordée, d'un côté, par un promoteur de plante et, de l'autre côté, par une séquence poly A d'une manière efficace pour permettre une expression de la séquence dans un hôte de type plante, et qui (ii) code une enzyme S-adénosylméthioninehydrolase qui hydrolyse de la S-adénosylméthionine en homosérine et 5'-méthylthioadénosine.

**2.** Vecteur suivant la revendication 1, caractérisé en ce que le promoteur de plante bordant la seconde séquence d'ADN procure une expression de la seconde séquence d'ADN qui est spécifique d'un tissu, dépendante de la température ou dépendante de la lumière.

**3.** Vecteur suivant l'une des revendications 1 et 2, caractérisé en ce que la seconde séquence d'ADN code la séquence de protéine présentée dans la figure 6.

**4.** Vecteur suivant l'une des revendications 1 et 2, caractérisé en ce que la seconde séquence d'ADN est dérivée d'un bactériophage choisi parmi le groupe comprenant le bactériophage T3 d'*Escherichia coli,* le coliphage BA14, le phage K11 de *Klebsiella* et le phage IV de *Seratti.*

**5.** Vecteur suivant l'une quelconque des revendications 1 à 4, caractérisé en ce que le gène utilisable pour une sélection génétique dans des cellules de plante confère une résistance à la kanamycine.

**6.** Vecteur suivant l'une quelconque des revendications 1 à 5, caractérisé en ce que le promoteur de plante bordant la seconde séquence d'ADN est un promoteur de synthétase de nopaline constitutif.

**7.** Vecteur suivant l'une quelconque des revendications 1 à 5, caractérisé en ce que le promoteur de plante bordant la seconde séquence d'ADN est un promoteur d'expression constitutif.

**8.** Vecteur suivant la revendication 7, caractérisé en ce que le promoteur d'expression constitutif est un promoteur du virus de la mosaïque du chou-fleur.

**9.** Procédé de réduction de biosynthèse d'éthylène dans des cellules de plantes, comprenant : une transformation de cellules hôtes de plante par un vecteur suivant l'une quelconque des revendications 1 à 8, procédé dans lequel les cellules hôtes de plante transformées sont capables d'exprimer ladite enzyme.

**10.** Procédé suivant la revendication 9, caractérisé en ce que la transformation d'un hôte de type plante est effectuée par une méthodologie de transformation directe choisie parmi le groupe comprenant une transformation de vecteur binaire par l'intermédiaire d'Agrobacterium, une électroporation, une microinjection et un bombardement par des microprojectiles.

**11.** Procédé suivant l'une des revendications 9 et 10, caractérisé en ce que l'hôte de type plante est *Nicotiania tabacum* L. cv. Wisconsin.

**12.** Procédé suivant l'une des revendications 9 et 10, caractérisé en ce que l'hôte de type plante est une tomate.

**13.** Cellule de plante transgénique contenant une séquence d'ADN qui code et exprime une enzyme S-adénosylméthioninehydrolase, où ladite enzyme peut hydrolyser de la S-adénosylméthionine en homosérine et 5'-méthylthioadénosine.

**14.** Cellule de plante transgénique suivant la revendication 13, comprenant en outre un gène utilisable pour une sélection génétique dans des cellules de plantes.

**15.** Cellule de plante transgénique suivant l'une des revendications 13 et 14, caractérisée en ce que la cellule de plante est obtenue à partir d'un fruit, d'un légume ou d'une plante produisant des fleurs.

FIG.1a

FIG.1b

FIG.1c

METHIONINE RECYCLING PATHWAY

GLUTAMINE

$\alpha$-KETOGLUTARATE

ATP

3pi

METHIONINE

S-ADENOSYLMETHIONINE

2-KETO-4-METHYLTHIOBUTYRATE

HCOOH

Pi

1-PHOSPHO-2,3-DIKETO-5-METHYLTHIOPENTANE

ACC

$C_2H_4$

5'-MEYHYLTHIOADENOSINE

MTA NUCLEOSIDASE

Ade

5-METHYLTHIORIBOSE-1-PHOSPHATE

MTR KINASE

ATP

5-METHYLTHIORIBOSE

EP 0 505 440 B1

FIG.2

FIG.3

EP 0 505 440 B1

FIG.4

FIG.5

```
                                                              XhoII
                                                              Sau3AI
                                                              | NlaIV
                                            TaqI           NdeII
                                       SalI    XbaI        MboI
                                       | MnlI|             CpfI
                           HindIII           PstI|HinfI    BstI
                           EcoVIII    SphI    |  | HincII
           AluI    NlaIII   | AluI   NlaIII   |  | AccI  | MaeI  BamHI
           |       |        | |      |        |  | |||   | |     | |
     1     ACAGCTATGACCATGATTACGCCAAGCTTGCATGCCTGCAGGTCGACTCTAGAGGAT     57
```

```
                 NcoI                    MboII  Xmn I        RsaI
                 |                       |      Asp700       |
     58    CCGCCACCATGGTTTTCACTAAAGAGCCTGCGAACGTCTTCTATGTACTGGTTTCCG     114
                 METValPheThrLysGluProAlaAsnValPheTyrValLeuValSerA
```

```
                     MnlI    MnlI         HphI              NlaIII
                     |       |            |                 |
     115   CTTTCCGTTCTAACCTCTGCGATGAGGTGAATATGAGCAGACACCGCCACATGGTAAGCA     174
           laPheArgSerAsnLeuCysAspGluValAsnMETSerArgHisArgHisMETValSerT
```

```
                 ScrFI
                 NciI                                  ScrFI
                 MspI                                  NciI
                 HpaII                     HinfI       MspI
           Fnu4HI |                NlaIV   | HincII    HpaII  MnlI
           |      ||               |       | |         ||     |
     175   CTTTACGTGCCGCACCGGGTCTTTATGGCTCCGTTGAGTCAACCGATTTGACCGGGTGCT     234
           hrLeuArgAlaAlaProGlyLeuTyrGlySerValGluSerThrAspLeuThrGlyCysT
```

```
               MnlI
               HinPlI
               | HhaI
               | CfoI   DdeI              RsaI
               | ||     |                 |
     235   ATCGTGAGGCAATCTCAAGCGCACCAACTGAGGAAAAAACTGTTCGTGTACGCTACAAGG     294
           yrArgGluAlaIleSerSerAlaProThrGluGluLysThrValArgValArgTyrLysA
```

# FIG. 6

```
                 HinPlI
                 |  Hhal
                 |  | Fnu4HI                   MaeI
                 |  | |                        |
                 |  | | CfoI        BbvI       | AluI
                 |  | | |           |          | |
   295  ACAAAGCGCAGGCACTCAATGTTGCACGCCTAGCTTGTAATGAGTGGGAGCAAGATTGCG  354
        spLysAlaGlnAlaLeuAsnValAlaArgLeuAlaCysAsnGluTrpGluGlnAspCysV


        RsaI   AccI         HinfI                    RsaI          TaqI
        |      |            |                        |             |
   355  TACTGGTATACAAATCACAGACTCACACGGCTGGTCTGGTGTACGCTAAAGGTATCGACG  414
        alLeuValTyrLysSerGlnThrHisThrAlaGlyLeuValTyrAlaLysGlyIleAspG


                           ScrFI                          HinPlI
                           NclI                           |  Hhal
                           MspI                   NlaIV   |  Cfol
                           HpaII     MnlI         |  BbvI |  BbvI  Fnu4HI
                           ||        |            |  |    |  |     |
   415  GGTATAAGGCTGAACGTCTGCCGGGTAGTTTCCAAGAGGTTCCTAAAGGCGCACCGCTGC  474
        lyTyrLysAlaGluArgLeuProGlySerPheGlnGluValProLysGlyAlaProLeuG


        Fnu4HI                          Fnu4HI      RsaI
        |                               |           |
   475  AAGGCTGCTTCACTATTGATGAGTTCGGTCGCCGCTGGCAAGTACAATAAGTGTTA  531
        lnGlyCysPheThrIleAspGluPheGlyArgArgTrpGlnValGln***
```

```
                                                     TaqI
                                                     SstI
                                          RsaI       SacI
                                  Sau3AI   NlaIV      HgiAI
                                  NdeII    | KpnI       EcoRI
                                  MboI     BanI|        BanII
        SfaNI  NlaIII             CpfI     Asp718   AluI| |
        |      |                  |        | | |    | | |
   532  AACTCAAGGTCATGCACGATGCGTGGCGGATCGGGTACCGAGCTCGAATTCACTGG  586
```

# FIG. 6 (cont.)